(19) Europäisches Patentamt
European Patent Office
Office européen des brevets

(11) Veröffentlichungsnummer : **0 135 152 B1**

# (12) EUROPÄISCHE PATENTSCHRIFT

(45) Veröffentlichungstag der Patentschrift : **07.06.89**

(51) Int. Cl.⁴ : **C 07 D513/18** // (C07D513/18, 341:00, 285:00, 285:00)

(21) Anmeldenummer : **84109745.4**

(22) Anmeldetag : **16.08.84**

## (54) Bis-[2,5-dithio - 1,3,4-thiadiazol] und Verfahren zu seiner Herstellung.

(30) Priorität : **27.08.83 DE 3330919**

(43) Veröffentlichungstag der Anmeldung : **27.03.85 Patentblatt 85/13**

(45) Bekanntmachung des Hinweises auf die Patenterteilung : **07.06.89 Patentblatt 89/23**

(84) Benannte Vertragsstaaten : **AT BE CH DE FR GB IT LI LU NL SE**

(56) Entgegenhaltungen :
**DE-C- 81 431**
**DE-C- 958 650**
**US-A- 3 087 932**
**US-A- 3 663 561**
**JOURNAL FÜR PRAKTISCHE CHEMIE, Band 60, 1899, Seiten 40-55, Leipzig; E. ZIEGELE "Über Thiobiazoldithiol"**
**Die Akte enthält technische Angaben, die nach dem Eingang der Anmeldung eingereicht wurden und die nicht in dieser Patentschrift enthalten sind.**

(73) Patentinhaber : **RHEIN-CHEMIE RHEINAU GMBH Postfach 81 04 09 Mülheimer Strasse 24-28 D-6800 Mannheim 81 (DE)**

(72) Erfinder : **Hugo, Peter, Prof. Dr. c/o Technische Universität**
**Strasse des 17. Juni 135**
**D-1000 Berlin 12 (DE)**
Erfinder : **Noack, Rainer, Dr.**
**Schillerstrasse 70**
**D-1000 Berlin 12 (DE)**

(74) Vertreter : **Jochum, Axel et al**
**c/o BAYER AG Zentralbereich Patente, Marken und Lizenzen**
**D-5090 Leverkusen 1, Bayerwerk (DE)**

**EP 0 135 152 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Bis-[2,5-dithio. -1,3,4-thiadiazol], im folgenden mit BDTD bezeichnet, und ein Verfahren zur Herstellung von BDTD aus 2,5-Dimercapto1,3,4-thiadiazol, im folgenden mit DMTD bezeichnet, und Wasserstoffperoxid.

BDTD ist ein wertvolles Zwischenprodukt zur Herstellung öllöslicher Korrosionsschutzzusätze.

Die Herstellung des Ausgangsstoffes DMTD ist aus Ber. 27, 2507 (1894) und DE-C-81 437 bekannt (s. Parallelanmeldung EP-A-135151).

Oxidationsreaktionen von DMTD sind bereits in der Literatur beschrieben. E. Ziegele, J. prakt. Chem. 60, 40 (1899) oxidiert alkoholische Lösungen von DMTD mit Jod oder Eisen (III) chlorid. In beiden Fällen identifiziert er die Verbindung der Formel :

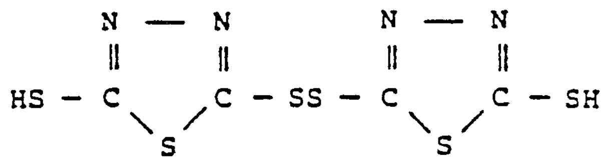

Mit Eisen (III) chlorid entsteht daneben ein unlöslicher Rückstand, der wahrscheinlich der Formel

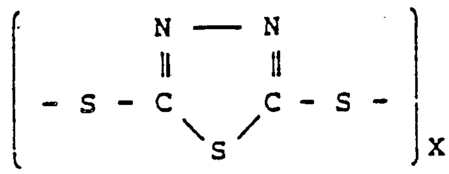

mit unbekannten Polymerisationsgrad X entspricht.

S. M. Losanitch in Soc. 121, 1542 (1923) führt die Oxidation einer alkoholischen Lösung von DMTD mit einem Überschuß von Jod durch. Er erhält eine Verbindung, die nach seinen Angaben nicht identisch mit der von Ziegele, l. c., gefundenen Polyverbindung ist und aufgrund der Elementaranalyse die Zusammensetzung $C_4H_4N_4S_7$ hat.

Eine Oxidation von DMTD mit $H_2O_2$ ist bisher nur in Anwesenheit weiterer Reaktionspartner beschrieben worden. Insbesondere werden in den US-Patenten 3 087 932 und 3 663 561 Verfahren beschrieben, bei denen durch gleichzeitigen Umsatz von DMTD, Wasserstoffperoxid und Mercaptanen direkt öllösliche Derivate des DMTD erhalten werden. Diese Produkte sind jedoch nicht einheitlich und bedürfen teilweise einer nachträglichen Aufarbeitung.

Der Erfindung liegt die Aufgabe zugrunde BDTD herzustellen.

Diese Aufgabe wird erfindungsgemäß dadurch gelöst, daß man DMTD und Wasserstoffperoxid in Abwesenheit weiterer Reaktionspartner bei Temperaturen bis 100 °C, vorzugsweise 20 bis 50 °C, umsetzt. Vorzugsweise wird DMTD als Aufschlämmung in Wasser eingesetzt, da DMTD in Wasser nur schwer löslich ist (ca. 10 g/l bei 20 °C). Das Molverhältnis von DMTD zu $H_2O_2$ beträgt bevorzugt 1 :1. Man erhält BDTD ohne störende Nebenprodukte.

Vorteilhaft ist weiterhin, daß das nach DE-C-958 650 zur Herstellung von DMTD anfallende Reaktionsgemisch ohne vorherige Isolierung dieses Stoffes nach dem erfindungsgemäßen Verfahren direkt zum BDTD weiterverarbeitbar ist. Dadurch werden Verluste durch die Isolierung des DMTD vermieden.

BDTD fällt mit praktisch quantitativer Ausbeute als in der Reaktionslösung unlöslicher, fast farbloser Niederschlag an. Die Elementaranalyse des gewaschenen und getrockneten Niederschlages gibt ein Atomverhältnis C :N :S = 2 :2 :3. Die massenspektroskopische Analyse ergibt das für BDTD geltende Molekulargewicht 296 und die nach der Strukturformel zu erwartenden Bruchstücke. Andere physikalische Molekulargewichtsbestimmungen sind wegen der Unlöslichkeit von BDTD in allen gebräuchlichen Lösungsmitteln nicht möglich.

### Beispiel 1

15 g DMTD (0,1 mol) wurden als Pulver mit einer Korngröße kleiner als 0,5 mm Durchmesser in 200 ml Wasser bei 20 °C suspendiert. Unter starkem Rühren wurden 12 g einer 35-%igen Wasserstoffperoxidlösung (entspricht 0,1 mol) so zugegeben, daß die Reaktionstemperatur 50 °C nicht überschritt. Eine Stunde nach Zugabe des Wasserstoffperoxides wurde BDTD abfiltriert und getrocknet. Die Ausbeute war mit 15 g (0,05 mol) quantitativ.

Beispiel 2

50 g (1 mol) Hydrazinhydrat und 120 g (3 mol) Natriumhydroxid in 500 ml Wasser wurden vorgelegt. Unter Kühlung und starkem Rühren wurden 152 g (2 mol) Schwefelkohlenstoff so zudosiert, daß die Reaktionstemperatur 40 °C nicht überstieg. Es wurde dann noch 3 Stunden bei 40 °C weitergerührt. Mit 300 g (3 mol) Salzsäure, 35-%ig wurde das DMTD ausgefällt, wobei gleichzeitig Schwefelwasserstoff entwich. Durch Begasen mit Stickstoff wurde der Schwefelwasserstoff vollständig ausgetrieben. Dem anfallenden Reaktionsgemisch wurde unter starkem Rühren 120 g einer 35-%igen Wasserstoffperoxidlösung (1 mol) so zudosiert, daß die Reaktionslösung 50 °C nicht überstieg. Eine Stunde nach Zugabe des Wasserstoffperoxides wurde BDTD abfiltriert und mit Wasser mehrfach gewaschen und getrocknet. Die Ausbeute an BDTD betrug 140 g, 93 % d. Th. bezogen auf Schwefelkohlenstoff und Hydrazinhydrat.

**Patentansprüche**

1. Bis-[2,5-dithio -1,3,4-thiadiazol] der Formel

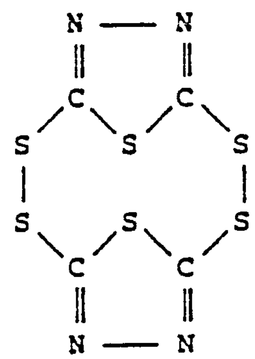

2. Verfahren zur Herstellung der Verbindung nach Anspruch 1, dadurch gekennzeichnet, daß man 2,5-Dimercapto-1,3,4-thiadiazol und Wasserstoffperoxid in Abwesenheit weiterer Reaktionspartner bei Temperaturen bis zu 100 °C miteinander umsetzt.

3. Verfahren nach Anspruch 2, dadurch gekennzeichnet, daß 2,5-Dimercapto-1,3,4-thiadiazol als Aufschlämmung in Wasser vorgelegt und Wasserstoffperoxid oder eine wäßrige Lösung von Wasserstoffperoxid zudosiert wird.

4. Verfahren nach Anspruch 2, dadurch gekennzeichnet, daß das Molverhältnis von 2,5-Dimercapto-1,3,4-thiadiazol zu $H_2O_2$ 1 :1 und die Umsetzungstemperatur 20 bis 50 °C betragen.

**Claims**

1. Bis-[2,5-dithio-1,3,4-thiadiazole] corresponding to the following formula

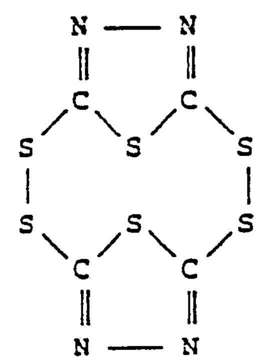

2. A process for the preparation of the compound claimed in claim 1, characterized in that 2,5-dimercapto-1,3,4-thiadiazole and hydrogen peroxide are reacted with one another in the absence of further reactants at temperatures of up to 100 °C.

3. A process as claimed in claim 2, characterized in that hydrogen peroxide or an aqueous solution of hydrogen peroxide is added to a suspension of 2,5-dimercapto-1,3,4-thiadiazole in water.

4. A process as claimed in claim 2, characterized in that the molar ratio of 2,5-dimercapto-1,3,4-thiadiazole to $H_2O_2$ is 1 : 1 and the reaction temperature is in the range from 20 to 50 °C.

**Revendications**

1. Le bis-[2,5-dithio-1,3,4-thiadiazole] de formule

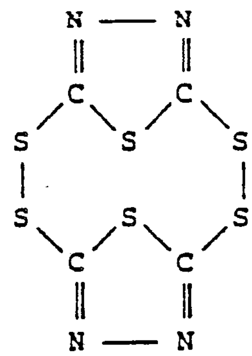

2. Procédé de production du composé suivant la revendication 1, caractérisé en ce qu'on fait réagir ensemble le 2,5-dimercapto-1,3,4-thiadiazole et du peroxyde d'hydrogène en l'absence d'autres partenaires réactionnels à des températures allant jusqu'à 100 °C.

3. Procédé suivant la revendication 2, caractérisé en ce que le 2,5-dimercapto-1,3,4-thiadiazole est préalablement introduit sous forme de suspension dans l'eau, et du peroxyde d'hydrogène ou une solution aqueuse de peroxyde d'hydrogène y est ajoutée.

4. Procédé suivant la revendication 2, caractérisé en ce que le rapport molaire du 2,5-dimercapto-1,3,4-thiadiazole au peroxyde d'hydrogène est égal à 1 : 1 et la température de réaction s'élève à 20-50 °C.